## Europäisches Patentamt
## European Patent Office
## Office européen des brevets

(11) Publication number: **0 132 038**
**A1**

(12)
# EUROPEAN PATENT APPLICATION

(21) Application number: **84303828.2**

(22) Date of filing: **06.06.84**

(51) Int. Cl.⁴: **A 01 N 35/02**
**A 01 N 25/06, A 61 L 2/22**

---

(30) Priority: **09.06.83 GB 8315880**

(43) Date of publication of application:
**23.01.85 Bulletin 85/4**

(84) Designated Contracting States:
**AT BE CH DE FR IT LI NL SE**

(71) Applicant: **RIZLA LIMITED**
**Severn Road Treforest Estate**
**Pontypridd Mid-Glamorgan CF37 5SP Wales(GB)**

(72) Inventor: **Minshull, Brian Sylvester**
**High Meadows Bettws Road Brynmenin**
**Nr. Bridgend Mid-Glamorgan Wales(GB)**

(72) Inventor: **Hayden, Jack**
**39 Oaken Grove**
**Maidenhead Berkshire(GB)**

(74) Representative: **Cole, Paul Gilbert et al,**
**Hughes Clark Andrews & Byrne 63 Lincoln's Inn Fields**
**London WC2A 3JU(GB)**

---

(54) Improvements in sprayable germicide materials.

(57) A spray container is filled with a virucidal spray composition comprising at least one volatile virucidal aldehyde, a volatile odour-masking perfume and a volatile organic solvent. Evidence has been found that the composition can kill herpes virus on surfaces onto which it has been sprayed.

EP 0 132 038 A1

## IMPROVEMENTS IN SPRAYABLE GERMICIDE MATERIALS

This invention relates to sprayable solutions having virucidal  and optionally also bactericidal activity on substrates onto which they are sprayed.  They are especially, though not exclusively, intended for the disinfection of toilet seats, though they are also suitable for the disinfection of other surfaces that may carry infection.

The prevalence of viral diseases such as herpes has become a matter of public concern,  and people may be worried about contraction of the disease from toilets used by the public because the herpes II virus has  life on an inert substrate of about 4 hours before it dies.  It is an object  of  the  invention  to  provide a  sprayable disinfectant formulation that can be carried around and sprayed onto the substrate, and is virustatically or virucidally effective while drying without noticeable residue within an appropriate short time period.

Accordingly the invention provides a spray container in which is a virustatically or a virucidally effective spray composition that when sprayed as a fine spray onto an impermeable substrate dries rapidly without visible residue and comprises at least one volatile virucidal material, a volatile odour-masking perfume material and a volatile organic solvent.

The compositions of the invention will preferably dry to the eye in a period of 30-90 seconds when sprayed onto a non-absorbent substrate such as a lavatory seat and notwithstanding this short wet contact time are surprisingly effective against herpes II virus. A sufficient residue of the active constituents remains after drying to provide anti-viral protection for up to 4 hours after spraying. Furthermore the compositions of the invention kill or inhibit the growth of Staphylococcus aureus, Escherichia coli, Pseudomonas-aeruginosa, Streptococcus faecalis, Neisseria gonorrhoea, Salmonella typhimurium, Candida albicans and Trichomonas vaginalis. Furthermore, because they also include a compatible volatile perfume material the compositions of the invention perfume and freshen the air where they are used.

The aldehydes that are incorporated into the above mentioned composition include formaldehyde which is preferred either for use alone or in admixture with a minor proportion of other aldehydes, but there could also be used as active ingredients acetaldehyde, propionaldehyde and other volatile n-alkyl aldehydes, glutaraldehyde, succinaldehyde, succinic dialdehyde, and other volatile aldehydes with antiviral activity, eg. aldehydes derived from essential oils such as citral. The concentrations of the active ingredients required will depend on the particular materials used. It is preferred, however, that at least two aldehyde materials should be present because they exhibit synergistic antiviral activity (O.Thraenhart and E. Kuvert, Zentralbl Bakteriol [Orig B]; Dec.75; 161 (3), 209-232). Formulations containing a major proportion of formaldehyde and a minor proportion of glutaraldehyde are preferred from the standpoints of virucidal effectiveness and volatility. A disinfectant formulation containing formaldehyde and glutaraldehyde is known under the name Tegodor, but that material also contains a quaternary ammonium surfactant that has residual activity. Such a material would not be acceptable for the present purpose because it would leave stains behind, and moreover it is intended to be applied diluted in water.

The volatile odour-masking material should share the volatility of the remainder of the composition, ie. it should substantially have vanished after 30-90 seconds. Materials that satisfy this criterion include materials having a lemon or lime odour such as lemon balm oil (which has also been reported to have antiviral activity, see L.S. Kucera et al, Ann N.Y. Acad. Sci. 130, 474-82 [1965]) terpeneless lemon oil, citral and essential oils derived from other species of the citrus family.

The volatile organic solvent may be methanol or ethanol, n-propanol, or isopropanol. Where, as is preferred, the solvent comprises a major proportion of ethanol it may be in admixture with minor amounts of water and with denaturants such as acetone, methyl ethyl ketone or a bittering agent such as Bitrex (denatronium benzoate). Bitrex may be present in denatured ethanol in an amount of about 10 micrograms per millilitre which is too low to leave a significant residue.

The liquid may be filled into the final container of choice using conventional filling equipment and is designed to be applied to inanimate objects as a fine spray. This can be achieved by means of a pump or aerosol actuator fitted to a glass, metal or plastics container. A lockable actuator for the container is also desirable and it is preferred that the spray should be directional.

The invention will now be further described by way of illustration in the following Examples.

4   0132038

Example 1

A concentrate was made up by mixing together the following materials (parts by volume):

Formaldehyde solution (37% w/v)........................54

Glutaraldehyde (25% w/v)..............................40

Terpeneless lemon oil BPC.............................2

Methylated spirits qsp...............................100ml

From the above concentrate samples were made up by dilution of the concentrate with the following quantities of methylated spirits:

| Identity | ml concentrate/100ml |
|---|---|
| A | 10 |
| B | 5 |
| C | 2.5 |
| D | 0.5 |
| E | 0.2 |
| F | 0.1 |

The above six formulations were filled into a pump action spray bottle and were effective against virus on lavatory seats as described below.

Example 2

The pH of the above formulations was adjusted to 8-8.5 with imidazole or other compatible alkaline material, after which the formulations were introduced into aerosol spray packs using carbon dioxide as propellant. The packed material exhibited satisfactory storage stability by reason of the carbon dioxide present in the container. But when the contents were sprayed onto a substrate, the sprayed material was alkaline, and effective against virus thereon.

Example 3

The above mentioned formulations were adjusted to pH 5 and were packed into an aerosol container using dichlorodifluoromethane as propellant.

Example 4

The following tests were carried out to demonstrate the activity of the formulations of Example 1:

(a)    <u>Spraying onto an impermeable surface</u>

The virus employed was a locally isolated strain of herpes virus hominis (HVH) type 2 which was grown in a monolayer culture of MRC 5 cells to a titre of 10 5.5 TCD 50.    The MRC cells (Flow Laboratories Limited) had been grown in Eagles MEM (Wellcome Reagents Ltd) supplemented by 10% calf serum which was used for all maintenance (M.M.).    The transport medium employed was Hanks solution with 10% bovine albumen.

For the tests, 20 oz screw copper sterile glass "replicate" bottles (medical flats) were rinsed with 1% Tween 20, drained and allowed to dry.    Into sets of five of the replicate bottles was placed 0.1 ml of the herpes virus hominis culture of TCD 50.    Then sample materials A-F either neat or diluted as indicated below were sprayed into each bottle using 4 puffs of a Roger's ENT spray (1 puff was about 0.04 ml of solution).    The bottles were left at room temperature for 10 minutes, after which the sprayed surface was swabbed and the swab was placed in a bottle containing 1 ml of transport medium.    Control bottles were inoculated with human herpes virus in the same way but were sprayed with sterile saline. They were then after-treated as described above.

0.2 ml of transport medium from the bottles under test were introduced into each of five tubes containing 1.0 ml of the culture of MRC 5 cells. After 4 hours the transport medium was replaced with 1.0 ml of fresh maintenance medium.    The cells were examined daily for a week for evidence of growth. The table below indicates for each test the number of tubes outof 25 for which there was evidence of the live virus:

| Formulation | Neat | 1/10 | 1/100 | 1/1000 |
|---|---|---|---|---|
| A | 0/25 | 0/25 | 0/25 | 6/25 |
| B | 0/25 | 0/25 | 7/25 | 18/25 |
| C | 0/25 | 0/25 | 14/25 | 21/25 |
| D | 0/25 | 0/25 | 18/25 | 22/25 |
| E | 0/25 | 4/25 | 20/25 | 23/25 |
| F | 0/25 | 15/25 | 21/25 | 25/25 |
| Saline control | 25/25 | – | – | – |

(b) <u>Spraying onto an absorbent material</u>

Pieces of sterile filter paper 2 cm square were inoculated with 0.1 ml of the 100 TCD 50 herpes virus hominis and dried at 37°C for 40 minutes. The inoculated pieces were sprayed with 0.16 ml of the formulations A-F and left for 10 minutes. They were then placed into 1 ml of maintenance medium, after which the maintenance medium was inoculated into the culture of MRC 5 cells. The maintenance medium was replaced as before for 4 hours and the tubes were examined daily for seven days for evidence of growth. The table below indicates for each test the number of tubes out of 25 for which there was evidence of live virus:

| Formulation | Neat | 1/10 | 1/100 | 1/1000 |
|---|---|---|---|---|
| A | 0/25 | 0/25 | 0/25 | 15/25 |
| B | 0/25 | 0/25 | 14/25 | 23/25 |
| C | 0/25 | 0/25 | 18/25 | 24/25 |
| D | 0/25 | 0/25 | 22/25 | 25/25 |
| E | 0/25 | 10/25 | 22/25 | 25/25 |
| F | 0/25 | 21/25 | 25/25 | 25/25 |
| Saline control | 25/25 | – | – | – |

(c) <u>Activity against selected bacteria, fungi and protozoa</u>

The activity of formulations A-F against the tabulated organisms was evaluated by the method of L.S. Stuart and L.F. Ortenzio, A.O.A.C. Journal, <u>58</u>, No.1, p.132,

Ref.3 (1975) and the results are as indicated, NG
indicating no growth of the test organism and Gr
indicating growth thereof:

Ref.3 (1975) and the results are as indicated, NG indicating no growth of the test organism and Gr indicating growth thereof:

| ORGANISM | A1 | A2 | A3 | B1 | B2 | B3 |
|---|---|---|---|---|---|---|
| Staphylococcus aureus | NG | NG | Gr | Gr | Gr | Gr |
| Escherichia coli | NG | NG | Gr | Gr | Gr | Gr |
| Pseudomonas aeruginosa | NG | Gr | Gr | Gr | Gr | Gr |
| Neisseria gonorrhoea | NG | NG | NG | Gr | Gr | Gr |
| Salmonella typhimurium | NG | Gr | Gr | Gr | Gr | Gr |
| Candida albicans | NG | Gr | Gr | Gr | Gr | Gr |

Conclusion

The formulations A-F were tested as described above against samples of herpes virus hominis on a hard non-absorbent surface simulating a lavatory seat and also on an absorbent surface simulating towelling and toilet paper. The formulations were very efficient at killing the virus both when neat and when further diluted. Activity has also been found against bacteria, fungi and protozoa.

EXAMPLE 5

EVALUATION OF THE VIRUCIDAL EFFICACY OF ANTIVIRAL SPRAYS R2/1/013 and "LYSOL" AGAINST HERPES VIRUS HOMINIS TYPE 2

A recently isolated strain of Herpes virus hominis (Herpes simplex virus Type 2) (HGV) derived from a case of genital herpes, was grown in tissue culture to a titre of $10^7$/ml, and was diluted to 1000 TCD$_{50}$/ml in PBS. The virus inoculum was sprayed over 7.5 x 10cm glass slides and over 2.5 cm$^2$ pieces of filter paper. Approximately 0.5 ml of inoculum was used for the slides and 0.1 ml for the papers. The 24 slides and 24 pieces of filter paper were dried and then sprayed for one second with the antiviral material A of Example 1 (called "AVS") and the same number for the same time with "Lysol" spray. The samples were kept at 18°C for various periods (5, 15, 45

8 0132038

and 120 minutes).

The glass surfaces were rubbed with cotton swabs moistened in tissue culture growth medium containing 40% calf serum. The swabs were placed in 2 ml of the same medium which after agitation was centrifuged at 50,000g in a "Spinco" refrigerated ultracentrifuge for two hours. The deposit was reconstituted with tissue culture maintenance medium and inoculated into a series of Vero Cell cultures. Six tubes were used for each slide. Cultures were examined daily for two weeks before being declared negative.

The filter paper squares were macerated in tissue culture media with 40% calf serum and the resultant fluid was treated as that from the slides. Slides and paper sprayed with virus but not with antiviral sprays were used as controls. A similar experiment was done but this time treating the slides only with antiviral spray before adding HSV. The treated slides were kept in batches of twelve for periods of 5, 15, 45 and 24 hours at 18°C. HSV was sprayed on as before and sampling was made after 5, 15 minutes and one hour. Untreated slides were used as controls. Further treatment was as described previously.

The results are set out in the following tables:-

GLASS SLIDES TREATED WITH HSV

| TIME | 5 minutes | 15 minutes | 45 minutes | 120 minutes |
|---|---|---|---|---|
| AVS | 0/6 | 0/6 | 0/6 | 0/6 |
| Lysol | 0/6 | 0/6 | 0/6 | 1/6 |
| Controls | 6/6 | 6/6 | 6/6 | 5/6 |

/6 represents number of positive cultures for HSV

FILTER PAPERS TREATED WITH HSV

| TIME | 5 minutes | 15 minutes | 45 minutes | 120 minutes |
|---|---|---|---|---|
| AVS | 0/6 | 0/6 | 0/6 | 0/6 |
| Lysol | 0/6 | 0/6 | 0/6 | 0/6 |
| Controls | 6/6 | 6/6 | 5/6 | 4/6 |

9

0132038

GLASS SLIDES TREATED WITH AVS

| TIME | 5 minutes | 15 minutes | 45 minutes | 24 hours |
|------|-----------|------------|------------|----------|
| 5 mins | 0/6 | 0/6 | 0/6 | 0/6 |
| 15 mins | 0/6 | 0/6 | 0/6 | 0/6 |
| 60 mins | 0/6 | 0/6 | 0/6 | 0/6 |
| Controls | 6/6 | 6/6 | 5/6 | 3/6 |

GLASS SLIDES TREATED WITH LYSOL

| TIME | 5 minutes | 15 minutes | 45 minutes | 24 hours |
|------|-----------|------------|------------|----------|
| 5 mins | 0/6 | 0/6 | 1/6 | 2/6 |
| 15 mins | 0/6 | 0/6 | 0/6 | 0/6 |
| 60 mins | 0/6 | 0/6 | 0/6 | 0/6 |
| Controls | 6/6 | 6/6 | 6/6 | 4/6 |

Both antiviral sprays were effective in killing HSV when used at a highly infectious dose under varying practical conditions. The first series of experiments showed that both sprays killed HSV which may be present on a hard or soft surface. However, although all the AVS had disappeared to the eye it was unexpectedly found in this experiment that the effects of the AVS material according to the invention were more persistent than that of "Lysol". The second series of experiments showed that virus was effectively killed when the impervious surface had been pretreated for periods up to a day. The AVS appeared to have a more long lasting action than Lysol.

These tests were made extremely difficult by the extreme toxicity of "Lysol" in particular for tissue culture cells. A preliminary experiment showed that when "Lysol" spray was diluted 1/10 in saline before use it became virtually non virucidal. This did not happen with AVS.

EXAMPLE 6

A virucidal composition was made up by mixing together the following ingredients:

|  | % w/v |
|---|---|
| Formaldehyde | 2.0 |
| Glutaraldehyde | 1.0 |
| Acetone | 5.0 |
| Distilled Water | 7.0 |
| Rosey Citrus (Florasynth 49662) | 0.1 |
| IMS | qsp 100 |

The above composition was charged into aerosol spray containers with carbon dioxide and a liquid non-substituted hydrocarbon as propellant. They were sprayed onto an inert surface of plastics material 20 cms square. The surface used for the purposes of the test was a formica laminate and the test area was sprayed in orthogonal directions to produce an even saturated film over the test area. These conditions enable an evaporation rate to be measured, but represent a significantly higher coverage rate than would be expected in normal use. The results obtained were as follows:

| Propellant | Amount dispensed | Drying time | Still Air Drying Rate 23°C |
|---|---|---|---|
| Carbon Dioxide | 1.4 g | 5.5 mins | 4mg/s |
| Hydrocarbon propellant (upright spray) | 1.2 g | 2 mins | 10mg/s |
| Hydrocarbon propellant (inverted spray valve) | 1.7 g | 3.5 mins | 9mg/s |

In the above tests the inverted spray was carried out using an aerosol container fitted with an appropriately adapted spray valve without dip tube. Such aerosol containers are available commercially.

It will be noted that the drying rate obtained with a liquid hydrocarbon propellant was more than twice the comparable rate using carbon dioxide as propellant. The reason is believed to be that the hydrocarbon propellant gives rise to a smaller drop size that enables a continuous liquid film to be developed at lower weights of composition per unit area.

[Note that the amount dispensed includes both the concentrate and the propellant and the proportion of propellent in the $CO_2$-propelled sample was much higher than that in the hydrocarbon-propelled sample.]

The above three samples were sprayed onto the surface again to produce a film that sparingly covered but did not saturate the surface. In each instance drying to the eye occurred in about 1 minute.

CLAIMS

1. A spray container for spraying contents onto a surface, said container being filled with a virucidally effective sprayable composition that includes a volatile virucidal material and a volatile organic solvent, characterised in that:

(a) the composition consists substantially wholly of volatile materials and dries to the eye rapidly and without visible residue under ambient conditions when sprayed onto an inert surface;

(b) the virucidal material is selected from the group consisting of formaldehyde, acetaldehyde, propionaldehyde, glutaraldehyde, succinaldehyde, succinic dialdehyde and virucidal essential oil derived aldehydes such as citral; and

(c) a volatile odour-masking material is present.

2. A container according to Claim 1, wherein the composition when sprayed in just sufficient amount to cover the surface with a thin continuous film dries under ambient conditions in 30-90 seconds.

3. A container according to Claim 2, wherein the aldehyde consists of a mixture of formaldehyde and glutaraldehyde.

4. A container according to any preceding claim, wherein the volatile odour-masking material comprises terpeneless lemon oil, lemon balm oil or citral.

5. A container according to any preceding claim, wherein there is present as solvent aqueous ethanol.

6. A container according to Claim 5, wherein the aqueous ethanol has been denatured with a minor proportion of acetone or methyl isobutyl ketone and with denatronium benzoate.

7. A container according to any preceding claim, further containing a liquid hydrocarbon as propellant for the composition.

8. A container according to any preceding claim, further containing carbon dioxide as propellant for the

2        **0132038**

composition, the pH of the composition being 8-8.5 and carbon dioxide being used a propellant.

9.      A container according to any preceding claim, wherein either (a) the pH of the composition is 5 and a hydrocarbon or halogenated hydrocarbon is used as propellant or (b) the propellant is compressed air from a pump fitted to the container.

10.      A method of disinfecting a surface which comprises spraying on to the surface contents of a container as claimed in any of Claims 1-9.

## DOCUMENTS CONSIDERED TO BE RELEVANT

| Category | Citation of document with indication. where appropriate, of relevant passages | Relevant to claim | CLASSIFICATION OF THE APPLICATION (Int. Cl. ³) |
|---|---|---|---|
| X | DR. B. HELWIG: "Moderne Arzneimittel", 5th edition, 1980, pages 588,589, Wissenschaftliche Verlagsgesellschaft mbH, Stuttgart, DE; * pages 588,589, "Buraton 25" and "Inciman Spray" * | 1,3,5, 10 | A 01 N 35/02 A 01 N 25/06 A 61 L 2/22 |
| Y | IDEM | 4-9 | |
| X | FR-A-1 354 159 (M. GATTEFOSSE et al.) * examples 1,2 * | 1,4,7 | |
| X | CHEMICAL ABSTRACTS, vol. 95, no. 17, 26th October 1981, page 110, no. 144580y, Columbus, Ohio, US; W. NICKLAS et al.: "Studies on the usefulness of different disinfectants for the aerosol disinfection of surfaces" & ZENTRALBL. BAKTERIOL., MIKROBIOL. HYG., ABT. 1, ORIG. B 1981, 173(5), 365-73 * abstract * | 1,10 | **TECHNICAL FIELDS SEARCHED (Int. Cl. ³)**<br><br>A 01 N A 61 L |
| Y | IDEM | 3 | |
| Y | DE-A-2 647 671 (DÖLLING) * pages 2,4; claim 1 * | 7,8 | |
| | --- -/- | | |

The present search report has been drawn up for all claims

| Place of search<br>THE HAGUE | Date of completion of the search<br>21-09-1984 | Examiner<br>PELTRE CHR. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

# European Patent Office

**EUROPEAN SEARCH REPORT**

**0132038**
Application number

EP 84 30 3828

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y | US-A-4 272 528  (A.M. VON ESCH et al.)<br>* column 4, lines 33-41 *<br><br>----- | 9 | |

**TECHNICAL FIELDS SEARCHED (Int. Cl. ³)**

The present search report has been drawn up for all claims

| Place of search | Date of completion of the search | Examiner |
|---|---|---|
| THE HAGUE | 21-09-1984 | PELTRE CHR. |